# EUROPEAN PATENT SPECIFICATION

(11) **EP 3 849 477 B1**
(45) Date of publication and mention of the grant of the patent: **30.10.2024**
(21) Application number: 18779918.4
(22) Date of filing: 12.09.2018
(51) Int. Cl.: A61F 2/95, A61F 2/966

(54) **LOW PROFILE DELIVERY SYSTEM WITH LOCK WIRE LUMEN**
NIEDRIGPROFILIGES ABGABESYSTEM MIT VERRIEGELUNGSDRAHTLUMEN
SYSTÈME D'ADMINISTRATION À BAS PROFIL AVEC LUMIÈRE DE FIL-FREIN

(43) Date of publication of application: 21.07.2021
(60) Divisional application: 24197747.9
(73) Proprietor: W. L. Gore & Associates, Inc., Newark, DE 19711 (US)
(72) Inventor: BURKART, Dustin C., Newark, Delaware 19711 (US)
(74) Representative: HGF
(86) International application number: PCT/US2018/050773
(87) International publication number: WO 2020/055403

(56) References cited:
- EP-A1- 2 777 643
- WO-A2-2009/042789
- US-A1- 2013 245 742

## Description

### FIELD

The present invention relates to medical devices suitable for treating an anatomical space (e.g., vessels) of the body, and to methods of manufacturing said medical devices. More specifically, the invention relates to apparatuses, and systems that include an implantable medical device prosthesis that allows for accurate deployment in the anatomical space, and to methods of manufacturing said apparatuses and systems.

### BACKGROUND

Disease of the vasculature is increasingly common. Treatment of the vasculature may be difficult because of the tortuous nature and complexity of the vasculature. Aortic dissections, for example, commonly begin at or near the aortic valve root and continue to the ascending aorta and the aortic arch. Medical devices implanted at a diseased state may be used for treatment of aortic dissections, aneurysms, and other diseases of the vasculature or other luminal systems of the body, such as the biliary tract, gastrointestinal tract, or respiratory system, for example. EP 2 777 643 is directed to a delivery device for deploying an expandable, endoluminal prosthesis within a body vessel. The delivery device may include one or more stent graft retention scaffolds having a plurality of arches, each arch encompassing an aperture forming a helical path and a trigger wire passing through at least two of the apertures following the helical path, wherein at least a portion of the stent graft is retained in a compressed configuration on the delivery device by the retention scaffold and trigger wire.

WO 2009/042789 is directed to a stent system comprising a stent body. At least one barb extends from the stent body and is configured such that a free end thereof is biased to extend radially outward from the stent body. A retaining mechanism is positioned to engage the at least one barb when the stent body is in a compressed state and retain the at least one barb in a tucked position relative to the stent body. A stent, a system for delivering a stent and a method of assembling a stent on a stent delivery shaft. The stent delivery system comprises a delivery shaft and a stent configured to be positioned about the delivery shaft. The stent includes an extension extending circumferentially from a portion of the stent to a free end, thereby defining a shoulder surface. The shoulder surface engages at least a portion of a belt to minimize axial movement of the belt during release of the release wire from engagement with the belt.

It remains desirable to provide medical devices, systems and methods for repairing disease along the aorta and also for repairing disease along the aorta and the branches extending and luminal spaces therefrom.

### SUMMARY

According to the invention, a medical device delivery system includes a catheter shaft; at least one loop attached to the catheter shaft; a medical device having an interior and an exterior; at least one opening through the medical device configured to allow the loop to extend from the interior to the exterior of the medical device; and a release line exterior to the medical device held in place by passing through the loop.

The theat least one loop is flexible and includes at least two flexible loops aligned relative to a longitudinal axis of the medical device.

The at least one flexible loop is configured to form a lumen for the release line.

According to the invention, a method of manufacturing a delivery system for a medical device includes coupling at least one flexible loop to a catheter shaft; arranging the at least one flexible loop through an opening in the medical device; and arranging a release line through the at least one flexible loop to releasably couple the medical device to the catheter shaft as further disclosed in claim 8.

### BRIEF DESCRIPTION OF THE DRAWINGS

The accompanying drawings are included to provide a further understanding of the disclosure and are incorporated in and constitute a part of this specification, illustrate embodiments, and together with the description serve to explain the principles of the disclosure.
FIG. 1 illustrates a side view of an example delivery system having a medical device in accordance with an embodiment.
FIG. 2 illustrates a perspective view of an example delivery system having a medical device in accordance with an embodiment.
FIG. 3 illustrates a perspective view of an example release line and a medical device in accordance with an embodiment.
FIG. 4 illustrates a side view of an example release line and a catheter shaft in accordance with an embodiment.
FIG. 5 illustrates a close-up view of an example flexible loop, a medical device, and a catheter shaft in accordance with an embodiment.
FIG. 6 illustrates a close-up view of an example flexible loop and a catheter shaft in accordance with an embodiment.
FIG. 7 illustrates a side view of an example release line, flexible loop, and a catheter shaft in accordance with an embodiment.

### DETAILED DESCRIPTION

Persons skilled in the art will readily appreciate that various aspects of the present disclosure can be realized by any number of methods and apparatus configured to perform the intended functions. It should also be noted that the accompanying drawing figures referred to herein are not necessarily drawn to scale, but may be exaggerated to illustrate various aspects of the present disclosure, and in that regard, the drawing figures should not be construed as limiting.

Various aspects of the present disclosure are directed toward apparatuses, systems, and methods that include an implantable medical device that may be used in treatment of the vasculature or other luminal and non-luminal systems of the body. In some examples, the implantable medical device is delivered to the vasculature using a delivery system, such as a transcatheter delivery system. In terms of configurations for the implantable medical device, a variety of configurations are contemplated, and the implantable medical devices described herein may be substantially cylindrical, include a bifurcation, or have any of a variety of features. Further, the implantable medical devices may be configured to conform to the vasculature into which the implantable medical device is implanted, and have a profile that facilitates delivery of the implantable medical device using a minimally invasive procedure (e.g., via transcatheter techniques), and able to withstand forces and other stresses that occur once implanted in the vasculature.

The medical device may be collapsed to a delivery configuration for delivery and expanded at the target location. In certain instances, the medical device may be partially expanded (*e.g*., for steering or orienting) or remain otherwise coupled to the catheter shaft during deployment. To maintain coupling of the medical device to the catheter, the delivery system may include a release line coupled to the medical device and the catheter.

As compared to delivery systems without a structural aspect to releasably couple a medical device to a catheter shaft, the release line, in certain instances, can increase an overall delivery profile of the system due in part to a catheter lumen for the release line. To facilitate delivery of the medical device with a release line (or other similar device-to-catheter coupling), the systems discussed herein include a flexible loop or lumen through which the release line is arranged. As discussed in further detail below, the flexible loop or lumen facilitate delivery of the medical devices discussed herein.

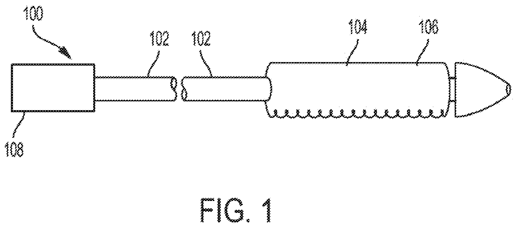

FIG. 1 illustrates a side view of an example delivery system 100 having a medical device 106 in accordance with an embodiment. The medical device 106 may be any endoluminal device suitable for delivery to the treatment area of a vasculature, or other luminal and non-luminal systems of the body (biliary tract, GI tract, respiratory system, or other as desired). As shown in FIG. 1, the delivery system 100 may also include a handle 108 at a proximal end of the system (the opposite end of the system at which the medical device 106 is arranged). The handle 108 of the delivery system 100 may be accessible to a user to assist in delivering the medical device 106. As explained in further detail below, the delivery system 100 may include one or more lines or wires that facilitate delivery, orientation, or placement of the medical device 106.

In various embodiments, the medical device 106 can include a radially collapsed configuration suitable for delivery to the treatment area of the vasculature of a patient. The medical device 106 can be constrained toward a radially collapsed configuration and releasably mounted onto a delivery device such as catheter shaft 102. The diameter of the medical device 106 in the collapsed configuration is small enough for the medical device 106 to be delivered through the vasculature to a target treatment location or area. In various embodiments, the diameter of the collapsed configuration is small enough to minimize the crossing profile of the delivery system 100 and reduce or prevent tissue damage to the patient. In the collapsed configuration, the medical device 106 can be guided by a catheter shaft 102 through the vasculature.

The medical device 106 can comprise a radially expanded configuration suitable for implanting the medical device 106 in the treatment area of a patient's vasculature. In the expanded configuration, the diameter of the medical device 106 can be approximately the same as the vessel or other body lumen in which the medical device 106 is to be placed. In other embodiments, the diameter of the medical device 106 in the expanded configuration can be slightly larger than the vessel to be treated to provide a friction or interference fit within the vessel.

In various embodiments, medical device 106 can comprise a self-expandable device, such as a self-expandable stent graft. Such devices dilate from a radially collapsed configuration to a radially expanded configuration when unrestrained. In other embodiments, medical device 106 can comprise a device that is expanded with the assistance of a secondary device applying an expansion force on the medical device 106, such as, for example, a balloon. In yet other embodiments, delivery system 100 can comprise a plurality of medical devices 106. The use of the delivery system 100 with any number of medical devices 106 is within the scope of the present disclosure. The medical devices 106 discussed herein may include, for example, stents, grafts, stent grafts, heart valves, and other similar devices. Thus, medical device 106 can include one or more stent components with one or more graft members disposed over and/or under the stent, which can dilate from a delivery diameter, through a range of larger intermediary diameters, and toward a maximal, pre-determined functional diameter. In certain instances, the medical device 106 includes one or more stent components made of nitinol and a graft member made of ePTFE. However, and as discussed below, any suitable combination of stent component(s) and graft member(s) is within the scope of the present disclosure.

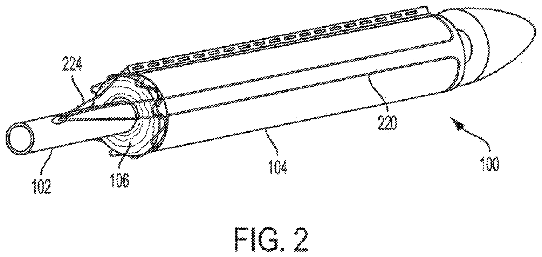

In certain instances, the delivery system 100 may include a sleeve or multiple sleeves (and other constrain mechanisms) to constrain the medical device 106 in a collapsed configuration for endoluminal delivery of the medical device 106 to a treatment portion of the vasculature or other body lumen of a patient. For the purposes of the disclosure, the term "constrain" may mean (i) to limit the expansion, either through self-expansion or assisted by a device, of the diameter of a medical device 106 or (ii) to cover or surround but not otherwise restrain a medical device 106 (e.g., for storage or biocompatibility reasons and/or to provide protection to the medical device 106 and/or the vasculature). The delivery system 100, for example, comprises a sleeve 104 (or pull-back sheath or other similar constraining structure) which surrounds and constrains medical device 106 toward a reduced diameter or collapsed configuration as shown in further detail with reference to FIG. 2.

FIG. 2 illustrates a perspective view of an example delivery system 100 having a medical device 106 in accordance with an embodiment. In various embodiments, the medical device 106 is constrained by a single sleeve 104 which circumferentially surrounds the medical device 106. In various embodiments, the sleeve 104 circumferentially surrounds the medical device 106 and constrains it toward a collapsed configuration, in which the diameter is less than the diameter of the medical device 106 in an unconstrained or otherwise deployed state or configuration. For example, the sleeve 104 may constrain the medical device 106 toward a collapsed configuration for delivery within the vasculature.

In other embodiments, the medical device 106 is constrained by a plurality of sleeves (e.g., similar to the sleeve 104) which circumferentially surround the medical device 106, which allow the medical device 106 to be deployed and held at intermediate configurations larger than the collapsed configuration and smaller than the deployed configuration. The plurality of sleeves can comprise at least two sleeves (e.g., each similar to sleeve 104) which circumferentially surround each other.

The sheet of material(s) used to form the sleeve(s) can comprise a series of openings, such that the openings extend from one edge of the sheet to the other. In such configurations, a coupling member 224 can be woven or stitched through the series of openings in the sheet of material(s), securing each of the two edges together and forming a tube. For example, in FIG. 2, the coupling member 224 secures the edges of sleeve 104 such that sleeve 104 maintains medical device 106 toward a reduced diameter or outer peripheral dimension suitable for endoluminal delivery.

In various embodiments, disengaging a single coupling member which closes a single sleeve from the sleeve allows the expandable device to be expanded toward a larger diameter or outer peripheral dimension. For example, with reference to FIG. 2, the delivery system can be used to deliver the medical device 106 to a treatment area of a vasculature. The medical device 106 has a collapsed diameter for delivery, and sleeve 104 circumferentially surrounds the medical device 106 and is held closed by the coupling member 224. As described in more detail below, bending of the medical device 106 can be controlled prior to full expansion (e.g., at an intermediate diameter) to help facilitate delivery to the desired position. Once medical device 106 is in position relative to the treatment area, the coupling member 224 is disengaged from the sleeve 104 and the sleeve 104 is released, allowing medical device 106 to expand toward a larger diameter.

In such embodiments, the medical device 106 can be expanded from the collapsed configuration toward the intermediate configuration once the implant has been delivered near the treatment area of the vasculature of a patient. The intermediate configuration may, among other things, assist in properly orienting and locating the medical device 106 within the treatment area of the vasculature. In various embodiments, a medical device 106 can be concentrically surrounded by two sleeves having different diameters. In such configurations, a primary sleeve constrains the medical device 106 toward the collapsed configuration. Once the collapsed configuration sleeve is opened, a secondary sleeve constrains the medical device 106 toward the intermediate configuration similarly configured and arranged as the sleeve 104 (e.g., the secondary sleeve may also be held together released by a coupling member).

As noted above, the medical device 106 may be oriented at the treatment area of the vasculature. To orient the medical device 106, the delivery system 100 includes a steering line 220. Tension can be applied to the steering line 220 to displace the steering line 220 and bend the medical device 106. Bending the medical device 106 may, among other things, assist in travelling through curved or tortuous regions of vasculature. Bending the medical device 106 may also allow the medical device 106 to conform to curvatures in the vasculature, or other body lumens of a patient.

In certain instances, the delivery system 100 may include a handle 108 (as shown in FIG. 1) to which the coupling member 224 and the steering line 220 (or lines) are connected. The handle 108 may allow the user to manipulate the coupling member 224 and the steering line 220 outside of the patient.

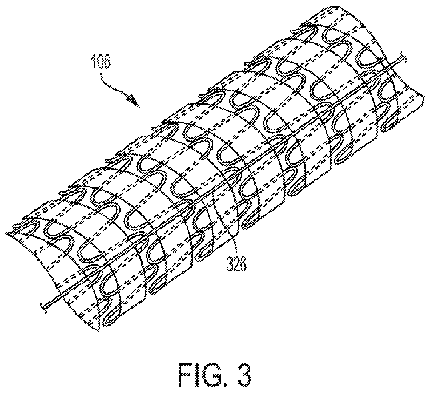

FIG. 3 illustrates a perspective view of an example release line 326 and a medical device 106 in accordance with an embodiment. A delivery system 100 may include a release line 326 to the medical device 106 to the delivery system 100. In addition, the release line 326 may also be used to secure a steering line 220 or multiple steering lines (e.g., each similar to another as desired) to the delivery system 100.

The release line 326 may be arranged external to the medical device 106 (as shown), may be internal to the medical device 106, or may be routed through the medical device 106 such that portions of the release line 326 are external to the medical device 106 and other portions of the release line 326 are internal to the medical device 106. Another end of the release line 326, similar to the coupling member 224 and steering line 220 discussed above, may be coupled to a handle 108 (not shown) of the delivery system 100. At the handle 108, tension may be applied to the release line 326 by a user to remove the release line 326 from the patient and uncouple the medical device 106 from the delivery system 100.

In various embodiments, the release line 326 can be formed from metallic, polymeric or natural materials and can comprise conventional medical grade materials such as nylon, polyacrylamide, polycarbonate, polyethylene, polyformaldehyde, polymethylmethacrylate, polypropylene, polytetrafluoroethylene, polytrifluorochlorethylene, polyvinylchloride, polyurethane, elastomeric organosilicon polymers; metals such as stainless steels, cobalt-chromium alloys and nitinol. Elongated members or lock wires can also be formed from high strength polymer fibers such as ultra high molecular weight polyethylene fibers (e.g., Spectra^{®}, Dyneema Purity^{®}, etc.) or aramid fibers (e.g., Technora^{®}, etc.).

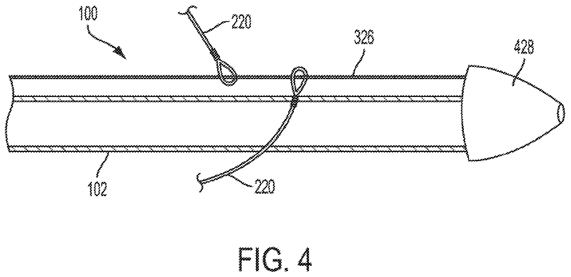

FIG. 4 illustrates a side view of an example release line 326 and a catheter shaft 102 in accordance with an embodiment. As shown in FIG. 4, a delivery system 100 incudes a catheter shaft 102, medical device 106, steering lines 220, and a release line 326. The release line 326 passes from outside of the body of the patient, from a handle 108 (not shown), through the catheter shaft 102, and exits distal to or near a distal end of the medical device 106. At this point, the release line 326 may extend along an exterior surface of the medical device 106.

In certain instances and as shown in FIG. 4, the release line 326 interacts with steering lines 220 continues to catheter tip 428. In such a configuration, the release line 326 releasably couples steering lines 220 to the delivery system 100. The release line 326 may be embedded, affixed, or attached to the catheter tip 428, and may be released from the catheter tip 428 in response to tension applied to the release line 326 by a user. Any manner in which the release line 326 may interact with steering line or lines 220 to maintain a releasable coupling between steering line or lines 220 and the delivery system 100 is within the scope of the present disclosure. Although two steering lines 220 are shown, it should be understood any number of steering lines 220 is contemplated, including fewer or greater than the two shown in FIG. 4.

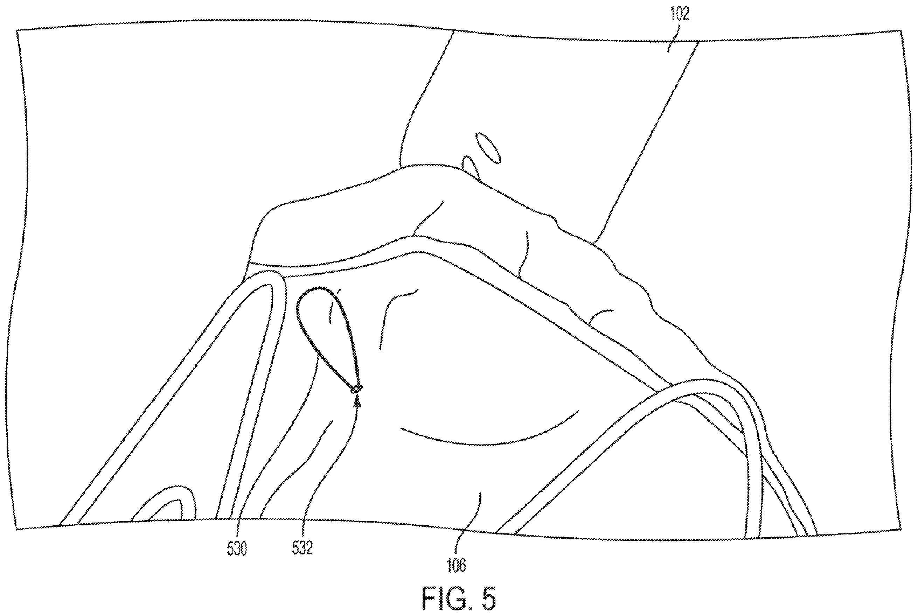

FIG. 5 illustrates a close-up view of a flexible loop 530, a medical device 106, and a catheter shaft 102 (that the medical device 106 is crushed or constrained onto) in accordance with an embodiment. As shown in FIG. 5, the medical device 106 includes an opening 532. The opening 532 through the medical device 106 may be configured to allow the flexible loop 530 to extend from an interior of the medical device 106 (through which the catheter shaft 102 is arranged) to an exterior of the medical device 106 as shown in FIG. 5. In certain instances, the opening 532 through the medical device 106 may be configured to allow the flexible loop 530 to extend from an exterior of the medical device 106 to an interior of the medical device 106.

The medical device 106 may be collapsed to a delivery configuration for delivery and expanded at the target location. In certain instances, the medical device 106 may be partially expanded (*e.g*., for steering or orienting) or remain otherwise coupled to the catheter shaft during deployment. To maintain coupling of the medical device 106 to the catheter shaft 102, the delivery system includes a release line 326 (as shown in FIGs. 3-4 and FIG. 7) coupled to the medical device 106 and the catheter shaft 102.

As compared to delivery systems without a structural aspect (e.g., a release wire, lock wire, or other similar structure) to releasably couple the medical device 106 to the catheter shaft 102, the release line 326, in certain instances, can increase an overall delivery profile of the system 100 due in part to the requirement for a catheter lumen to maintain the release line. To facilitate delivery of the medical device 106 with a release line (or other similar device-to-catheter coupling), a flexible loop 530 serves as a pathway for the release line 326. In certain instances, the delivery systems discussed herein may include multiple flexible loops 530. In certain instances, the flexible loop 530 (or flexible loops 530) may form a flexible lumen through which the release line 326 is arranged.

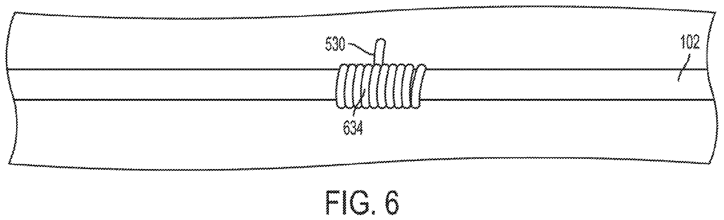

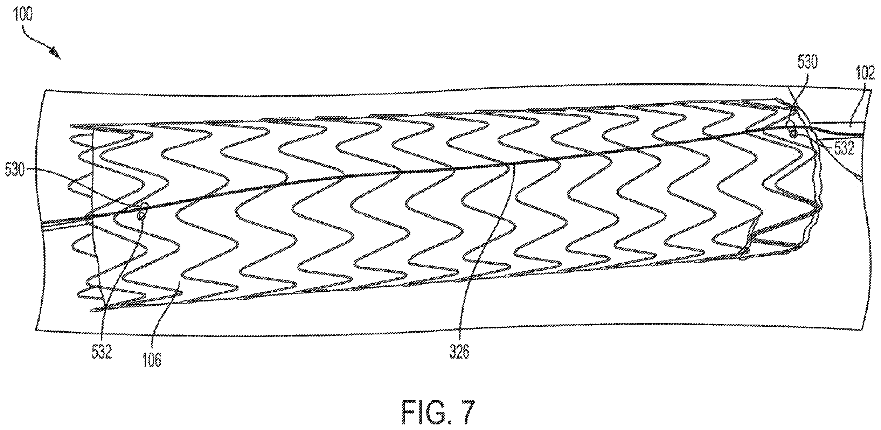

In certain instances, the flexible loop 530 (flexible loops 530) may be attached to the catheter shaft 102 (as shown in FIG. 6). The flexible loop 530 (flexible loops 530 or lumen) may be configured to collapse in response to reduction in diameter of the medical device 106. In this manner, the flexible loop 530 (flexible loops 530 or lumen) may facilitate delivery by forming a pathway or lumen for a release wire. Thus, the flexible loop 530 (flexible loops 530 or lumen) lessen a size or diameter of a catheter usable for the delivery systems discussed herein by forming a pathway for a release wire where a lumen of a catheter would previously been used. As shown in further detail in FIG. 7, the flexible loop 530 (flexible loops 530 or lumen) may hold the release line 326 or similar feature in place exterior to the medical device 106 by being passed through the flexible loop 530.

FIG. 6 illustrates a close-up view of an example flexible loop 530 and a catheter shaft 102 in accordance with an embodiment. As shown in FIG. 6, the flexible loop 530 is coupled to the catheter shaft 102. The flexible loop 530 may be attached to the catheter shaft 102 along a portion of the catheter shaft 102 that extends through a lumen of a medical device, such as the medical device 106.

In certain instances, the flexible loop 530 includes a fiber 634 coupled to the catheter shaft 102. The fiber 634 may be wrapped, glued, bonded, sewn, tacked, interlocked or otherwise coupled to the catheter shaft 102 to form the flexible loop 530. The medical device 106 (as discussed above but not shown) discussed herein may be a stent and graft combination, or any configuration described in association with the medical device 106. The flexible loop 530 may be formed of the same or a similar material as the graft material of the medical device. In addition, the opening through which the flexible loop 530 is arranged may be arranged through the graft portion of the medical device 106.

To manufacture a delivery system that includes the flexible loop 530 or more than one flexible loop 530, the flexible loop 530 is coupled to the catheter shaft 102 (*e.g.*, wrapping, gluing, bonding, sewing, tacking, interlocking, or otherwise coupling the fiber 634 to the catheter shaft 102 to form the flexible loop 530). After the flexible loop 530 is formed, the flexible loop 530 is arranged through an opening (*e.g*., as shown in FIG. 5) in the medical device 106. Subsequently, a release line may be arranged through the flexible loop 530 to releasably couple the medical device to the catheter shaft 102 (*e.g.*, as shown in FIG. 7). After the release line is arranged through the flexible loop 530, the medical device may be collapsed against the catheter shaft 102 to a delivery configuration, which can also include collapsing of the flexile loop 530 toward the catheter shaft 102 in response to the medical device being collapsed to the delivery configuration.

FIG. 7 illustrates a side view of an example release line 326, flexible loop 530, and a catheter shaft 102 in accordance with an embodiment. The release line 326, flexible loop 530, and catheter shaft 102 form portions of a delivery system 100. The medical device 106 has an interior and an exterior. As shown in FIG. 7, a portion of the catheter shaft 102 is arranged through the interior of the medical device 106, and the release line 326 is arranged across the exterior of the medical device 106.

As shown in FIG. 7, the delivery system 100 includes two flexible loops 530. According to the invention, the two flexible loops 530 are aligned relative to a longitudinal axis of the medical device 106, which may be aligned with the catheter shaft 102. Further, the medical device 106 is configured to collapse to a delivery configuration from an expanded/delivery configuration shown in FIG. 7. In these instances, the flexible loop 530 or flexible loops 530 are configured to collapse toward the catheter shaft 102 in response to the medical device 106 being collapsed to the delivery configuration. The delivery system 100 may include one or more sheaths/sleeves (*e.g*., as shown in FIGs. 1-2) configured to releasably hold the medical device 106 in the delivery configuration.

In certain instances, the flexible loop 530 (or loops 530) is configured to maintain at least a portion of the release line 326 in contact with an exterior surface of the medical device 106. Thus, the flexible loop 530 is configured to form a lumen for the release line 326 according to the invention. The flexible loop 530 (or flexible loops 530 as shown in FIG. 7) are attached to the catheter shaft 102 and extend through an opening 532 (or opening 532 for each respective flexible loop 530) through the medical device 106. The flexible loop 530 (or lumen) is configured to collapse in response to reduction in diameter of the medical device 106. In certain instances, the lumen is formed by a first loop 530 arranged at one of the medical device 106 and a second loop 530 arranged at another end of the medical device 106, with the release line 326 being arranged through the flexible lumen (formed by the loops 530). The release line 326 is configured to releasably couple the medical device 106 to the catheter shaft 102.

In certain instances and as shown in FIG. 4, the release line 326 may terminate at a catheter tip 428 (or olive) of the catheter shaft 102. The release line 326 is configured to release from the catheter tip 428 (or olive) in response to tension and withdraw through the flexible loop(s) 530. As noted above, the medical device 106 may be collapsed to a delivery configuration for delivery and expanded at the target location. In certain instances, the medical device 106 may be partially expanded (*e.g*., for steering or orienting) or remain otherwise coupled to the catheter shaft during deployment. To maintain coupling of the medical device 106 to the catheter shaft 102, the release line 326 is coupled to the medical device 106 by being arranged through the flexible loop(s) 530 (or lumen) and to the catheter shaft 102. Thus, the medical device 106 may be partially expanded and maintained in contact with the delivery system 100 and the catheter shaft 102 by way of the release line 326. The release line 326 and the loop(s) 539 are configured to maintain the medical device 106 coupled to the catheter shaft 102 during orientation of the medical device 106.

The flexible loop(s) 530, in certain instances, facilitate a minimal delivery profile of the system 100 due in part to removing the need for a lumen for the catheter lumen for the release line 326 (which can add to overall delivery size). In certain instances, the flexible loop 530 (or flexible loops 530) may form a flexible lumen through which the release line 326 is arranged, and which may collapse along with the medical device 106.

After orienting and deployment of the medical device 106, the release line 326 is configured to withdrawn through the flexible loop(s) 530 in response to tension. The flexible loop(s) 530 are fixedly coupled to the catheter shaft 106. The catheter shaft 102 may be removed from the vasculature after deployment of the medical device 106 and leave the medical device 106 at the target treatment location. The catheter shaft 102 may be withdrawn to remove the system 100 from the vasculature. By withdrawing the catheter shaft 102, the flexible loop(s) 530 are also removed due to the flexible loop(s) 530 being fixedly attached to the catheter shaft 102. The flexible loop(s) facilitate use of the release line 326 without increasing the size of the delivery system 100, which may facilitate use of the system 100 through reach into smaller vasculature as compared to prior systems.

Potential materials for graft members of the medical devices discussed herein include, for example, expanded polytetrafluoroethylene (ePTFE), polyester, polyurethane, fluoropolymers, such as perflouorelastomers and the like, polytetrafluoroethylene, silicones, urethanes, ultra-high molecular weight polyethylene, aramid fibers, and combinations thereof. Other embodiments for a graft member material can include high strength polymer fibers such as ultra-high molecular weight polyethylene fibers (e.g., Spectra^{®}, Dyneema Purity^{®}, etc.) or aramid fibers (e.g., Technora^{®}, etc.). The graft member may include a bioactive agent. In one embodiment, an ePTFE graft includes a carbon component along a blood contacting surface thereof. Any graft member which can be delivered by a catheter is in accordance with the present disclosure.

In addition, stent components of the medical devices discussed herein can have various configurations such as, for example, rings, cut tubes, wound wires (or ribbons) or flat patterned sheets rolled into a tubular form. Stent components can be formed from metallic, polymeric or natural materials and can comprise conventional medical grade materials such as nylon, polyacrylamide, polycarbonate, polyethylene, polyformaldehyde, polymethylmethacrylate, polypropylene, polytetrafluoroethylene, polytrifluorochlorethylene, polyvinylchloride, polyurethane, elastomeric organosilicon polymers; metals such as stainless steels, cobalt-chromium alloys and nitinol and biologically derived materials such as bovine arteries/veins, pericardium and collagen. Stent components can also comprise bioresorbable materials such as poly(amino acids), poly(anhydrides), poly(caprolactones), poly(lactic/glycolic acid) polymers, poly(hydroxybutyrates) and poly(orthoesters). Any expandable stent component configuration which can be delivered by a catheter is in accordance with the present disclosure.

The invention of this application has been described above both generically and with regard to specific embodiments. It will be apparent to those skilled in the art that various modifications and variations can be made in the embodiments without departing from the scope of the disclosure. Thus, it is intended that the embodiments cover the modifications and variations of this invention provided they come within the scope of the appended claims.

## Claims

1. A medical device delivery system (100), the system (100) comprising:
a catheter shaft (102);
at least one loop (530) attached to the catheter shaft (102);
a medical device (106) having an interior and an exterior;
at least one opening (532) through the medical device (106) configured to allow the loop (530) to extend from the interior to the exterior of the medical device (106); and
a release line (326) exterior to the medical device (106) held in place by passing through the loop (530),
wherein the at least one loop (530) is flexible and includes at least two flexible loops (530) aligned relative to a longitudinal axis of the medical device (106); and
wherein the at least one flexible loop (530) is configured to form a lumen for the release line (326).

2. The system (100) of claim 1, wherein the at least one flexible loop (530) includes a fiber (634) coupled to the catheter shaft (102); and optionally
wherein the fiber (634) is at least one of wrapped, glued, and bonded to the catheter shaft (102) to form the at least one flexible loop (530).

3. The system (100) of any one of claims 1-2, wherein the medical device (106) is configured to collapse to a delivery configuration and the at least one flexible loop (530) is configured to collapse toward the catheter shaft (102) in response to the medical device (106) being collapsed to the delivery configuration; and optionally
further comprising at least one sheath (104) configured to releasably hold the medical device (106) in the delivery configuration.

4. The system (100) of any one of claims 1-3, wherein the medical device (106) comprises a stent and graft combination and the at least one opening (532) is formed through the graft, and the at least one flexible loop (530) comprises a graft material.

5. The system (100) of any one of claims 1-4, wherein the at least one flexible loop (530) is configured to maintain at least a portion of the release line (326) in contact with an exterior surface of the medical device (106).

6. The system (100) of any one of claims 1-5, wherein the release line (326) terminates in an olive (428) at one end of the catheter shaft (102); and optionally
wherein the release line (326) is configured to release from the olive (428) in response to tension and withdraw through the at least one flexible loop (530).

7. The system (100) of any one of claims 1-6, wherein the release line (326) and the at least one loop (530) are configured to maintain the medical device (106) coupled to the catheter shaft (102) during orientation of the medical device (106).

8. A method of manufacturing a delivery system (100) for a medical device (106), the method comprising:
coupling at least two flexible loops (530) to a catheter shaft (102) aligned relative to a longitudinal axis of the medical device (106), wherein the at least two flexible loops (530) are configured to form a lumen for a release line (326);
arranging the at least two flexible loops (530) through an opening (532) in the medical device (106); and
arranging the release line (326) through the at least two flexible loops (530) to releasably couple the medical device (106) to the catheter shaft (102).

9. The method of claim 8, wherein coupling the at least two flexible loops (530) to the catheter shaft (102) includes at least one of wrapping, gluing, and bonding a fiber (634) to the catheter shaft (102) to form the at least two flexible loops (530).

10. The method of any one of claims 8-9, further comprising collapsing the medical device (106) against the catheter shaft (102) to a delivery configuration and collapsing the at least two flexile loops (530) toward the catheter shaft (102) in response to the medical device (106) being collapsed to the delivery configuration.

11. The system (100) of claim 1, wherein
the lumen is a flexible lumen attached to the catheter shaft (102) and extends through at least one opening (532) through the medical device (106), the flexible lumen being configured to collapse in response to reduction in diameter of the medical device (106); and
wherein the release line (326) is arranged through the flexible lumen and configured to releasably couple the medical device (106) to the catheter shaft (102).

12. The system (100) of claim 11, wherein the flexible lumen includes the first flexible loop (530) and the second flexible loop (530) arranged at opposite ends of the medical device (106); and optionally
wherein the release line (326) is configured to withdrawn through the first flexible loop (530) and the second flexible loop (530) in response to tension, and the first flexible loop (530) and the second flexible loop (530) maintain coupled to the catheter shaft (102); and optionally
wherein the catheter shaft (102) is configured to withdraw the first flexible loop (530) and the second flexible loop (530) after delivery of the medical device (106).

13. The system (100) of claim 11, wherein the at least one flexible loop (530) includes a fiber (634) coupled to the catheter shaft (102) and the fiber (634) is at least one of wrapped, glued, and bonded to the catheter shaft (102) to form the at least one flexible loop (530).

## Patentansprüche

1. System (100) zur Abgabe einer medizinischen Vorrichtung, wobei das System (100) Folgendes umfasst:
einen Katheterschaft (102);
zumindest eine Schlaufe (530), die an dem Katheterschaft (102) angebracht ist;
eine medizinische Vorrichtung (106) mit einem Inneren und einem Äußeren;
zumindest eine Öffnung (532) durch die medizinische Vorrichtung (106), die konfiguriert ist, um zu ermöglichen, dass sich die Schlaufe (530) von dem Inneren zu dem Äußeren der medizinischen Vorrichtung (106) erstreckt; und
eine Freigabeleitung (326) außerhalb der medizinischen Vorrichtung (106), die durch Verlaufen durch die Schlaufe (530) an Ort und Stelle gehalten wird,
wobei die zumindest eine Schlaufe (530) flexibel ist und zumindest zwei flexible Schlaufen (530) beinhaltet, die relativ zu einer Längsachse der medizinischen Vorrichtung (106) ausgerichtet sind; und
wobei die zumindest eine flexible Schlaufe (530) konfiguriert ist, um ein Lumen für die Freigabeleitung (326) zu bilden.

2. System (100) nach Anspruch 1, wobei die zumindest eine flexible Schlaufe (530) eine Faser (634) beinhaltet, die an den Katheterschaft (102) gekoppelt ist; und wobei optional die Faser (634) zumindest eines von an den Kathetherschaft (102) gewickelt, geklebt und gebunden ist, um die zumindest eine flexible Schlaufe (530) zu bilden.

3. System (100) nach einem der Ansprüche 1-2, wobei die medizinische Vorrichtung (106) konfiguriert ist, um in eine Abgabekonfiguration zusammenzuklappen und die zumindest eine flexible Schlaufe (530) konfiguriert ist, um als Reaktion darauf, dass die medizinische Vorrichtung (106) in die Abgabekonfiguration zusammengeklappt wird, zu dem Katheterschaft (102) zusammenzuklappen; und optional
ferner umfassend zumindest eine Hülle (104), die konfiguriert ist, um die medizinische Vorrichtung (106) lösbar in der Abgabekonfiguration zu halten.

4. System (100) nach einem der Ansprüche 1-3, wobei die medizinische Vorrichtung (106) eine Kombination aus Stent und Transplantat umfasst und die zumindest eine Öffnung (532) durch das Transplantat gebildet wird und die zumindest eine flexible Schlaufe (530) ein Transplantatmaterial umfasst.

5. System (100) nach einem der Ansprüche 1-4, wobei die zumindest eine flexible Schlaufe (530) konfiguriert ist, um zumindest einen Teil der Freigabeleitung (326) in Kontakt mit einer Außenfläche der medizinischen Vorrichtung (106) zu halten.

6. System (100) nach einem der Ansprüche 1-5, wobei die Freigabeleitung (326) in einer Olive (428) an einem Ende des Katheterschafts (102) endet; und optional
wobei die Freigabeleitung (326) konfiguriert ist, um sich als Reaktion auf Spannung und Rückzug durch die zumindest eine flexible Schlaufe (530) von der Olive (428) zu lösen.

7. System (100) nach einem der Ansprüche 1-6, wobei die Freigabeleitung (326) und die zumindest eine Schlaufe (530) konfiguriert sind, um die medizinische Vorrichtung (106) während Ausrichtung der medizinischen Vorrichtung (106) an den Katheterschaft (102) gekoppelt zu halten.

8. Verfahren zur Herstellung eines Abgabesystems (100) für eine medizinische Vorrichtung (106), wobei das Verfahren Folgendes umfasst:
Koppeln von zumindest zwei flexiblen Schlaufen (530) an einen Katheterschaft (102), der relativ zu einer Längsachse der medizinischen Vorrichtung (106) ausgerichtet ist, wobei die zumindest zwei flexiblen Schlaufen (530) konfiguriert sind, um ein Lumen für eine Freigabeleitung (326) zu bilden;
Anordnen der zumindest zwei flexiblen Schlaufen (530) durch eine Öffnung (532) in der medizinischen Vorrichtung (106); und
Anordnen der Freigabeleitung (326) durch die zumindest zwei flexiblen Schlaufen (530), um die medizinische Vorrichtung (106) lösbar an den Katheterschaft (102) zu koppeln.

9. Verfahren nach Anspruch 8, wobei das Koppeln der zumindest zwei flexiblen Schlaufen (530) an den Katheterschaft (102) zumindest eines von Wickeln, Kleben und Binden einer Faser (634) an den Katheterschaft (102) beinhaltet, um die zumindest zwei flexiblen Schlaufen (530) zu bilden.

10. Verfahren nach einem der Ansprüche 8-9, ferner umfassend Zusammenklappen der medizinischen Vorrichtung (106) gegen den Katheterschaft (102) in eine Abgabekonfiguration und Zusammenklappen der zumindest zwei flexiblen Schlaufen (530) zu dem Katheterschaft (102) als Reaktion darauf, dass die medizinische Vorrichtung (106) in die Abgabekonfiguration zusammengeklappt wird.

11. System (100) zur Abgabe einer medizinischen Vorrichtung nach Anspruch 1, wobei
das Lumen ein flexibles Lumen ist, das an dem Katheterschaft (102) angebracht ist und sich durch zumindest eine Öffnung (532) durch die medizinische Vorrichtung (106) erstreckt, wobei das flexible Lumen konfiguriert ist, um als Reaktion auf Reduzierung des Durchmessers der medizinischen Vorrichtung (106) zusammenzuklappen; und
wobei die Freigabeleitung (326) durch das flexible Lumen angeordnet und konfiguriert ist, um die medizinische Vorrichtung (106) lösbar an den Katheterschaft (102) zu koppeln.

12. System (100) nach Anspruch 11, wobei das flexible Lumen die erste flexible Schlaufe (530) und die zweite flexible Schlaufe (530) beinhaltet, die an gegenüberliegenden Enden der medizinischen Vorrichtung (106) angeordnet sind; und optional
wobei die Freigabeleitung (326) konfiguriert ist, um sich durch die erste flexible Schlaufe (530) und die zweite flexible Schlaufe (530) als Reaktion auf Spannung zurückzuziehen und die erste flexible Schlaufe (530) und die zweite flexible Schlaufe (530) an den Katheterschaft (102) gekoppelt bleiben; und optional
wobei der Katheterschaft (102) konfiguriert ist, um die erste flexible Schlaufe (530) und die zweite flexible Schlaufe (530) nach Abgabe der medizinischen Vorrichtung (106) zurückzuziehen.

13. System (100) nach Anspruch 11, wobei die zumindest eine flexible Schlaufe (530) eine Faser (634) beinhaltet, die an den Katheterschaft (102) gekoppelt ist, und die Faser (634) zumindest eines von an den Katheterschaft (102) gewickelt, geklebt und gebunden ist, um die zumindest eine flexible Schlaufe (530) zu bilden.

## Revendications

1. Système (100) de pose de dispositif médical, le système (100) comprenant :
une tige de cathéter (102) ;
au moins une boucle (530) fixée à la tige de cathéter (102) ;
un dispositif médical (106) comportant un intérieur et un extérieur ;
au moins une ouverture (532) à travers le dispositif médical (106) conçue pour permettre à la boucle (530) de s'étendre à partir de l'intérieur jusqu'à l'extérieur du dispositif médical (106) ; et
une ligne de libération (326) extérieure au dispositif médical (106) maintenue en place en passant à travers la boucle (530),
ladite au moins une boucle (530) étant souple et comprenant au moins deux boucles souples (530) alignées par rapport à un axe longitudinal du dispositif médical (106) ; et
ladite au moins une boucle souple (530) étant conçue pour former une lumière pour la ligne de libération (326).

2. Système (100) de la revendication 1, ladite au moins une boucle souple (530) comprenant une fibre (634) couplée à la tige de cathéter (102) ; et éventuellement, ladite fibre (634) étant au moins enveloppée, collée ou liée à la tige de cathéter (102) pour former la au moins une boucle souple (530).

3. Système (100) de l'une quelconque des revendications 1-2, ledit dispositif médical (106) étant conçu pour se replier dans une configuration de pose et ladite au moins une boucle souple (530) étant conçue pour se replier vers la tige de cathéter (102) en réponse au repliement du dispositif médical (106) dans la configuration de pose ; et éventuellement comprenant en outre au moins une gaine (104) conçue pour maintenir de manière amovible le dispositif médical (106) dans la configuration de pose.

4. Système (100) de l'une quelconque des revendications 1-3, ledit dispositif médical (106) comprenant une combinaison de stent et de greffon et ladite au moins une ouverture (532) étant formée à travers le greffon, et ladite au moins une boucle souple (530) comprenant un matériau de greffon.

5. Système (100) de l'une quelconque des revendications 1-4, ladite au moins une boucle souple (530) étant conçue pour maintenir au moins une partie de la ligne de libération (326) en contact avec une surface extérieure du dispositif médical (106).

6. Système (100) de l'une quelconque des revendications 1-5, ladite ligne de libération (326) se terminant par une olive (428) au niveau d'une extrémité de la tige de cathéter (102) ; et éventuellement, ladite ligne de libération (326) étant conçue pour se libérer de l'olive (428) en réponse à une tension et se retirer à travers la au moins une boucle souple (530).

7. Système (100) de l'une quelconque des revendications 1-6, ladite ligne de libération (326) et ladite au moins une boucle (530) étant conçues pour maintenir le dispositif médical (106) couplé à la tige de cathéter (102) durant l'orientation du dispositif médical (106).

8. Procédé de fabrication d'un système (100) de pose pour un dispositif médical (106),
le procédé comprenant :
le couplage d'au moins deux boucles souples (530) à une tige de cathéter (102) alignées par rapport à un axe longitudinal du dispositif médical (106), lesdites au moins deux boucles souples (530) étant conçues pour former une lumière pour une ligne de libération (326) ;
l'agencement des au moins deux boucles souples (530) à travers une ouverture (532) dans le dispositif médical (106) ; et
l'agencement de la ligne de libération (326) à travers les au moins deux boucles souples (530) pour coupler de manière amovible le dispositif médical (106) à la tige de cathéter (102).

9. Procédé de la revendication 8, ledit couplage des au moins deux boucles souples (530) à la tige de cathéter (102) comprenant au moins l'un de l'enroulement, du collage et de la liaison d'une fibre (634) à la tige de cathéter (102) pour former les au moins deux boucles souples (530).

10. Procédé de l'une quelconque des revendications 8-9, comprenant en outre le repliement du dispositif médical (106) contre la tige de cathéter (102) dans une configuration de pose et le repliement des au moins deux boucles souples (530) vers la tige de cathéter (102) en réponse au repliement du dispositif médical (106) dans la configuration de pose.

11. Système (100) de pose de dispositif médical selon la revendication 1, ladite lumière étant une lumière souple fixée à la tige de cathéter (102) et s'étendant à travers au moins une ouverture (532) à travers le dispositif médical (106), la lumière souple étant conçue pour se replier en réponse à une réduction du diamètre du dispositif médical (106) ; et ladite ligne de libération (326) étant agencée à travers la lumière souple et conçue pour coupler de manière amovible le dispositif médical (106) à la tige de cathéter (102).

12. Système (100) de la revendication 11, ladite lumière souple comprenant la première boucle souple (530) et la seconde boucle souple (530) agencées au niveau des extrémités opposées du dispositif médical (106) ; et éventuellement
ladite ligne de libération (326) étant conçue pour être retirée à travers la première boucle souple (530) et la seconde boucle souple (530) en réponse à une tension, et ladite première boucle souple (530) et ladite seconde boucle souple (530) étant maintenues couplées à la tige de cathéter (102) ; et éventuellement
ladite tige de cathéter (102) étant conçue pour retirer la première boucle souple (530) et la seconde boucle souple (530) après la pose du dispositif médical (106).

13. Système (100) de la revendication 11, ladite au moins une boucle souple (530) comprenant une fibre (634) couplée à la tige de cathéter (102) et ladite fibre (634) étant au moins un parmi enveloppée, collée et liée à la tige de cathéter (102) pour former la au moins une boucle souple (530).
